# EUROPEAN PATENT APPLICATION

(11) **EP 4 506 957 A1**
(43) Date of publication of application: **12.02.2025**
(21) Application number: 23796581.9
(22) Date of filing: 25.01.2023
(51) Int. Cl.: G16H 50/70, G16H 10/60, G16H 50/20, A61B 5/00, G06F 21/31, A61B 5/021, A61B 5/01, A61B 5/145, A61B 5/024

(54) **MEDICAL DATA TRANSMISSION METHOD AND SYSTEM THEREFOR**

(30) Priority: 25.04.2022 KR 20220050918
(71) Applicant: I-SENS, INC., Seoul 06646 (KR)
(72) Inventor: NAM, Hakhyun, Seoul 06509 (KR)
(74) Representative: HGF
(86) International application number: PCT/KR2023/001141
(87) International publication number: WO 2023/210929

(57) **Abstract**

The present disclosure provides a medical data transmission system for transmitting/receiving medical data, comprising: a user terminal for collecting first user medical data and transmitting first user identification information; and a data reception device which identifies the first user on the basis of the first user identification information received from the user terminal, and which receives preset data, based on the first user identification information, of the first user medical data collected by the user terminal, so as to output the preset data.

## Description

### Technical Field

The present disclosure relates to a method for transmitting and collecting medical data. More specifically, it relates to a system and method for directly transmitting medical data measured using a user terminal to a medical data reception device, wherein the medical data reception device outputs the corresponding information.

### Background Art

Recently, with the advancement of medical technology and healthcare, the number of individuals who continuously monitor their current health status and check their health status by linking the identified information with the medical system is increasing. This need is increasing the number of people pursuing 'wellness', which refers to a physically, mentally, and socially healthy state.

In addition, patients with specific diseases are monitoring their health by receiving health monitoring information such as blood pressure, heart rate, oxygen saturation, and blood sugar information in real time using various devices provided by medical institutions or medical equipment sales companies.

As the above technology has developed, the medical industry and the communication industry have recently been merged, so that individuals may obtain health information such as biosignals and body fluid components using their own smart devices (including mobile phones, smart watches, etc.), and the information is transmitted to the server of the application installed on the smart device and managed.

Meanwhile, in the case of patients with specific diseases such as hypertension and diabetes who require continuous monitoring, they are given a diet, have appropriate exercise opportunities, and are also given insulin therapy or oral medication, and therefore, it has become possible to obtain the patient's biometric data of in various ways using blood sugar sensors or blood sugar measurement devices attached to the body of diabetic patients in their daily lives, and the obtained biometric data is transmitted to a dedicated application of a smart device, and is then transmitted to the electronic medical record (EMR) of a university hospital through the application, allowing medical institutions to monitor the patient's condition.

However, in the case of monitoring a specific disease, patients may have different medical data to monitor depending on their physical condition, disease type, constitution, and other environmental factors, even if their symptoms are similar, but there was a problem in that the data transmitted from the application of the smart device did not reflect this because the medical data was uniformly provided from the server.

In addition, the obtained medical data is stored on the server by passing through the application, and the data reception device equipped in the medical institution receives the information stored on the server, so the individual's medical record is stored on an external server, leading to a risk of personal information leakage.

### Disclosure of the Invention

### Technical Goals

The present disclosure is to provide a system and method for directly transmitting specific information requested from a medical data reception device without going through a server or cloud using a user terminal that obtains medical data of a user.

### Technical Solutions

In an embodiment of the present disclosure, a medical data transmission system for transmitting and receiving medical data may include a medical data transmission system including a user terminal configured to collect first user medical data and transmit first user identification information, and a data reception device configured to identify a first user based on the first user identification information received from the user terminal, receive preset data based on the first user identification information among the first user medical data collected from the user terminal, and output the preset data.

In addition, the first user identification information may include personal information including at least one of age, gender, and name of the first user.

In addition, the first user medical data may include health information including at least one of blood pressure, body temperature, oxygen saturation, and heart rate, and blood sugar measurement information including at least one of maximum blood sugar, minimum blood sugar, average blood sugar, fasting blood sugar, specific time blood sugar information, pre-meal blood sugar information, post-meal blood sugar information, pre- and post-exercise blood sugar information, and a blood sugar graph according to a blood sugar monitoring method of the first user.

In addition, the preset data may be set based on diagnostic information corresponding to the first user identification information.

In addition, when the diagnostic information is 'average blood sugar monitoring required', the preset data may include at least one of maximum blood sugar, minimum blood sugar, average blood sugar, and a blood sugar graph among blood sugar measurement information of the first user.

In addition, when the diagnostic information is 'specific time blood sugar monitoring required', the preset data may include, among blood sugar measurement information of the first user, at least one of fasting blood sugar, specific time blood sugar information, pre-meal blood sugar information, post-meal blood sugar information, and pre- and post-exercise blood sugar information.

In addition, when the diagnostic information is 'blood pressure or body temperature monitoring required', the preset data may include at least one information of blood pressure, body temperature, oxygen saturation, and heart rate among health information of the first user.

In addition, the data reception device may be configured to update the preset data based on additional diagnostic information of the first user.

In addition, the data reception device may be configured to, when a distance from the user terminal is within a predetermined distance, communicate with the user terminal and identify the first user using any one of QR authentication, Wi-Fi, Bluetooth, P2P, NFC, and RFID.

In addition, the medical data transmission system may further include a server connected to the user terminal, and the user terminal may be configured to transmit the first user medical data to the server, and the first user medical data transmitted to the server and the preset data may be different from each other.

In addition, the preset data may correspond to data specified by a second user of the data reception device based on the first user identification information, first user diagnostic information, and second user identification information of the data reception device.

In addition, the second user identification information may include the second user identification information comprises at least one of identification information on a medical staff using the data reception device, information on medical institution providing medical services, and medical data request information additionally requested by a specific medical staff and a specific medical institution among the medical data.

In addition, the system may further include a blood sugar measurement device configured to measure the first user medical data and transmit the first user medical data to the user terminal, wherein the blood sugar measurement device includes a communication part for communicating with the user terminal, a blood sugar measurement part configured to measure blood sugar information of the first user, and a data generation part configured to generate data based on a type of the blood sugar measurement device.

In addition, a medical data transmission method for transmitting and receiving user medical data may include

by a user terminal, communicating with a data reception device and collecting first user medical data, by the data reception device, identifying the user terminal and a first user and receiving preset data based on first user identification information among the first user medical data collected from the user terminal, and outputting, by the data reception device, the preset data.

In addition, the preset data may correspond to data specified by a second user of the data reception device based on the first user identification information, first user diagnostic information, and second user identification information of the data reception device, and the second user identification information of the data reception device may include at least one of identification information on a medical staff using the data reception device, information on a medical institution providing medical services, and medical data request information additionally requested by a specific medical staff and a specific medical institution among the medical data.

### Advantageous Effects

According to various embodiments of the present disclosure, a user may experience personalized medical services by transmitting specific information requested from the data reception device among health information obtained from the user terminal to the data reception device.

In addition, when information transmitted to the data reception device is output, medical institution workers may use the data to grasp the health status of the user required for treatment at a glance, thereby reducing the effort required for treatment.

### Brief Description of Drawings

FIG. 1 illustrates a configuration diagram of a user terminal 100 according to an embodiment of the present disclosure.
FIG. 2 illustrates a configuration diagram of a data reception device 200 according to an embodiment of the present disclosure.
FIG. 3 illustrates a medical data transmission system 1 according to an embodiment of the present disclosure.
FIG. 4 is an operation flowchart illustrating a medical data transmission method according to an embodiment of the present disclosure.
FIG. 5 is a diagram illustrating medical data according to an embodiment of the present disclosure.
FIG. 6 is a diagram illustrating data for determining preset data according to an embodiment of the present disclosure.
FIG. 7 illustrates a medical data transmission system in which a smart device and a user terminal are connected according to an embodiment of the present disclosure.

### Best Mode for Carrying Out the Invention

Hereinafter, the embodiments disclosed in this specification will be described in detail with reference to the attached drawings, and regardless of the drawing symbols, identical or similar components will be given the same reference numerals and redundant descriptions thereof will be omitted. The suffixes 'module' and 'part' used for components in the following description are given or used interchangeably only for the convenience of writing the specification, and do not have distinct meanings or roles in themselves. In addition, when describing the embodiments disclosed in this specification, if it is determined that a specific description of a related known technology may obscure the gist of the embodiments disclosed in this specification, the detailed description thereof will be omitted. In addition, the attached drawings are only intended to facilitate easy understanding of the embodiments disclosed in this specification, and the technical ideas disclosed in this specification are not limited by the attached drawings, and should be understood to include all modifications, equivalents, and substitutes included in the spirit and technical scope of the present disclosure.

Terms including ordinal numbers such as first, second, etc., may be used to describe various components, but the components are not limited by the terms. The terms are used only for the purpose of distinguishing one component from another.

When a component is referred to as being 'connected' or 'coupled' to another component, it should be understood that it may be directly connected or coupled to the other component, but there may be other components in between. On the other hand, when a component is referred to as being 'directly connected' or 'directly coupled' to another component, it should be understood that there are no other components in between.

In addition, each component shown in the present disclosure is independently depicted to represent different characteristic functions, and does not mean that each component part is composed of separate hardware or a single software component.

In other words, each component part is listed and included as a separate component for convenience of explanation, and at least two component parts among each component part may be combined to form one component part, or one component part may be divided into multiple component parts to perform functions, and integrated embodiments and separate embodiments of each component part are also included in the scope of the present disclosure as long as they do not deviate from the essence of the present disclosure.

In addition, some components are not essential components that perform essential functions in the present disclosure, but may be optional components merely for improving performance. The present disclosure may be implemented by including only essential component parts for implementing the essence of the present disclosure, excluding components used only for performance improvement, and a structure including only essential components, excluding optional components used only for performance improvement, is also included in the scope of the present disclosure.

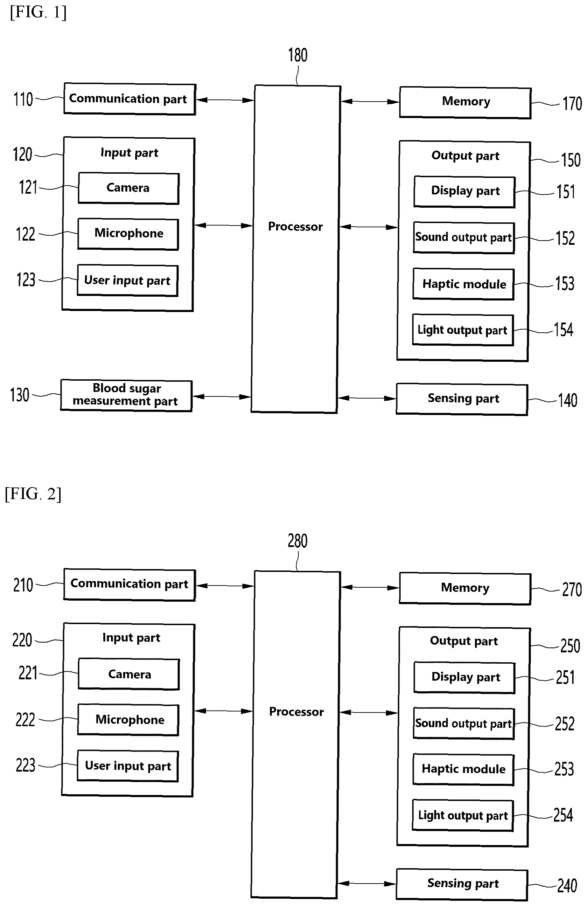

FIG. 1 illustrates a configuration diagram of a user terminal 100 according to an embodiment of the present disclosure.

The user terminal 100 may be implemented as a mobile device such as a mobile phone, a smart phone, a personal digital assistant (PDA), a portable multimedia player (PMP), a navigation, a tablet PC, a wearable device, etc.

Referring to FIG. 1, the user terminal 100 may include a communication part 110, an input part 120, a blood sugar measurement part 130, a sensing part 140, an output part 150, a memory 170, a processor 180, etc.

The communication part 110 may transmit and receive data with external devices such as other electronic devices or servers using wired or wireless communication technology. For example, the communication part 110 may transmit and receive sensor information, user input, control signals, etc., with external devices.

Here, the communication technology used by the communication part 110 includes global system for mobile communication (GSM), code division multi access (CDMA), long term evolution (LTE), 5G, wireless LAN (WLAN), wireless-fidelity (Wi-Fi), Bluetooth, radio frequency identification (RFID), infrared data association (IrDA), ZigBee, near field communication (NFC), etc.

The input part 120 may obtain various types of data.

At this time, the input part 120 may include a camera for inputting a video signal, a microphone for receiving an audio signal, a user input part for inputting information from a user, etc. Here, the camera or microphone may be treated as a sensor, and a signal obtained from the camera or microphone may be referred to as sensing data or sensor information.

The blood sugar measurement part 130 may measure the user's blood sugar level. The blood sugar measurement part 130 may include all known blood sugar measurement technologies, such as a patch type attached to the user's body and a needle type for directly collecting blood.

The sensing part 140 may obtain at least one of internal information of the user terminal 100, surrounding environment information of the user terminal 100, and user information using various sensors.

At this time, the sensors included in the sensing part 140 include a proximity sensor, an illuminance sensor, an acceleration sensor, a magnetic sensor, a gyro sensor, an inertial sensor, an RGB sensor, an IR sensor, a fingerprint recognition sensor, an ultrasonic sensor, a light sensor, a microphone, a lidar, a radar, etc.

The output part 150 may generate output related to vision, hearing, or touch, etc.

At this time, the output part 150 may include a display part that outputs visual information, a speaker that outputs auditory information, a haptic module that outputs tactile information, etc.

The memory 170 may store data that supports various functions of the user terminal 100. For example, the memory 170 may store input data obtained from the input part 120, various data obtained from a server 300 or a connected device.

The processor 180 may determine at least one executable operation of the user terminal 100. Then, the processor 180 may control the components of the user terminal 100 to perform the determined operation.

To this end, the processor 180 may request, search, receive, or utilize data of the memory 170, and control the components of the user terminal 100 to perform an operation that is predicted or an operation that is determined to be desirable among the at least one executable operation.

At this time, the processor 180 may, if the connection of an external device is required to perform the determined operation, generate a control signal for controlling the external device, and transmit the generated control signal to the external device.

The processor 180 may control at least some of the components of the user terminal 100 to drive an application program stored in the memory 170. Furthermore, the processor 180 may also operate two or more of the components included in the user terminal 100 in combination with each other to drive the application program.

FIG. 2 illustrates a configuration diagram of a data reception device 200 according to an embodiment of the present disclosure.

According to an embodiment of the present disclosure, the data reception device 200 may be a term referring to various devices that receive medical data. For example, the data reception device 200 should be interpreted as a concept including a healthcare and diagnostic device such as a medical terminal that receives medical data, a medical data management device equipped in a hospital or a medical institution, etc.

The data reception device 200 according to an embodiment of the present disclosure may communicate directly with the user terminal 100 and may be implemented as a fixed device or a movable device such as a TV, a projector, a mobile phone, a smart phone, a desktop computer, a laptop, a digital broadcasting terminal, a personal digital assistant (PDA), a portable multimedia player (PMP), a navigation, a tablet PC, a wearable device, a set-top box (STB), a DMB receiver, a radio, a washing machine, a refrigerator, a desktop computer, digital signage, a robot, a vehicle, etc.

Referring to FIG. 2, the data reception device 200 may include a communication part 210, an input part 220, a sensing part 240, an output part 250, a memory 270, a processor 280, etc., and each component may include the same function as the user terminal 100 illustrated in FIG. 1. Therefore, overlapping descriptions will be omitted.

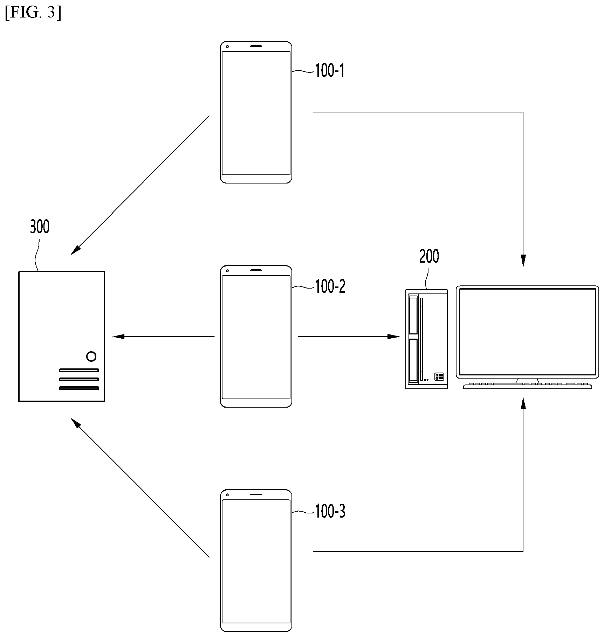

Hereinafter, a medical data transmission system for transmitting and receiving medical data of a user of a user terminal will be described with reference to FIG. 3.

The user of the user terminal according to an embodiment of the present disclosure may obtain various body information such as blood pressure, oxygen saturation, heart rate, and body temperature, and an institution providing medical services such as health checkups requires health monitoring of users of the user terminal.

In addition, patients with specific diseases such as hypertension and diabetes require continuous monitoring. In particular, in the case of diabetic patients, blood sugar levels should be measured at specific times or periodically using a blood sugar sensor or blood sugar measurement device attached to the body of diabetic patients in daily life, and medication appropriate for the blood sugar levels should be taken or a lifestyle guided by a medical institution should be followed. In addition, if the blood sugar level exceeds a certain level, they should visit the hospital immediately.

In the past, treatment was carried out by using a blood sugar measurement device that specializes in blood sugar measurement, or by transmitting data measured by the blood sugar measurement device linked to the blood sugar measurement device to a dedicated application of a smart device such as a mobile phone, and checking the data recorded in the dedicated application at a medical institution.

In the present disclosure, by using a medical data transmission system that transmits and receives the user's medical data, a medical data reception device is installed at a medical institution or a place where medical data is required, so that when the user approaches within a predetermined distance, the user's medical data may be immediately received, and the received medical data may be output using the display part of the data reception device, thereby providing convenience to the user of the medical data reception device. Hereinafter, it will be described in detail below.

FIG. 3 is a diagram illustrating a system according to an embodiment of the present disclosure.

First, before explaining the present disclosure, the user of the user terminal 100 will be referred to as a first user, and the user of the data reception device 200 will be referred to as a second user. At this time, the first user may mean a patient receiving medical services, a general public, a person undergoing a checkup, etc., and may include a diabetic patient, a hypertensive patient, etc., as specific diseases.

In addition, the second user should be understood as a concept including all medical staff providing medical services, workers at medical-related institutions, etc.

Referring to FIG. 3, the medical data transmission system for transmitting and receiving medical data of the first user according to an embodiment of the present disclosure may include at least one user terminal 100-1, 100-2, 100-3, a data reception device 200, and a server 300.

At this time, the medical data should be understood as a concept including health information including at least one of blood pressure, body temperature, oxygen saturation, and heart rate, and blood sugar measurement information including at least one of maximum blood sugar, minimum blood sugar, average blood sugar, fasting blood sugar, specific time blood sugar information, pre-meal blood sugar information, post-meal blood sugar information, pre- and post-exercise blood sugar information, and a blood sugar graph according to a blood sugar monitoring method.

According to an embodiment of the present disclosure, the user terminal 100 may be a smart device that collects the medical data of the first user and provides it to another device.

The user terminal may directly obtain the medical data of the first user by using a blood sugar measurement part or a sensor (e.g., a blood pressure sensor, a temperature sensor, a heart rate measurement sensor, a blood sugar measurement sensor, etc., capable of directly obtaining medical data of a diabetic patient) and sensing part capable of receiving various types of biometric data, and may also obtain medical data by linking with a separate blood sugar measurement device, application, and smart device.

The user terminal 100 may transmit the collected medical data to the server 300 and may receive data stored in the server 300. In addition, the user terminal may transmit specific data among the collected medical data to the data reception device 200.

The data reception device 200 according to an embodiment of the present disclosure may receive the medical data of the first user from the user terminal.

First, the data reception device 200 of the present disclosure may be a reception device for receiving medical data, and should be understood as a general term for equipment placed in a medical institution or placed in a place for collecting medical data.

The data reception device 200 according to an embodiment of the present disclosure may communicate with at least one user terminal 100-1, 100-2, 100-3. Each of the user terminals may be used by different users and may collect medical data of each of the different users.

For convenience, the symbol of the user terminal will be referred to as '100' hereinafter.

At this time, the data reception device 200 may communicate with the user terminal 100 using a short-range communication technology. Specifically, the communication parts of each of the data reception device 200 and the user terminal 100 may transmit and receive information using wireless-fidelity (Wi-Fi), Bluetooth, radio frequency identification (RFID), infrared data association (IrDA), QR code, near field communication (NFC), P2P service, etc.

The communication may be performed automatically when the distance between the user terminal and the data reception device is within a predetermined distance. Here, the predetermined distance may mean a distance corresponding to the coverage provided by the short-range communication technology.

The data reception device 200 according to an embodiment of the present disclosure may receive the medical data of the first user and output the received medical data through the display part.

According to an embodiment of the present disclosure, the medical data transmission system may include a server 300. The server 300 may be connected to the user terminal 100 and may receive medical data collected from the user terminal 100. Specifically, the server 300 may receive medical data using a medical data collection specified application installed on the user terminal 100 or may receive data stored in the memory equipped in the user terminal 100 itself.

Hereinafter, an operation flowchart of a medical data transmission system according to an embodiment of the present disclosure will be described.

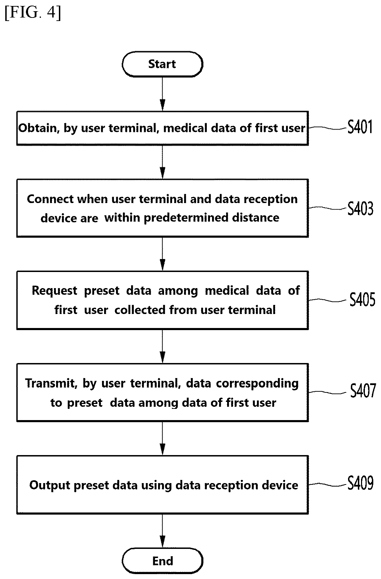

FIG. 4 is an operation flowchart of a medical data transmission system according to an embodiment of the present disclosure.

Referring to FIG. 4, the user terminal 100 may obtain the medical data of the first user (S401).

As described above, the user terminal may directly obtain the medical data of the first user, and may obtain the medical data by linking with a separate blood sugar measurement device or smart device. The collected data may be stored in the memory of the user terminal 100.

At this time, the medical data may include health information and blood sugar measurement information of the first user.

According to an embodiment of the present disclosure, when the user terminal and the data reception device are within a predetermined distance, the user terminal 100 and the data reception device 200 may be connected (S403).

Meanwhile, 'connection' may also mean direct connection using a cable connector, but it is preferable to be electrically connected using wireless communication technology.

In an embodiment, when the user terminal 100 and the data reception device 200 communicate using short-range communication, each of the user terminal and the data reception device may transmit a signal requesting a connection when the terminal to be communicated with comes within a communicable distance.

Both terminals may obtain first user identification information from the transmitted signal and identify the first user using the obtained first user identification information.

At this time, the first user identification information may include personal information including at least one of the age, gender, and name of the first user using the user terminal 100, unique information of the user terminal 100, and communication identification information of the user terminal 100 and the data reception device 200.

For example, when using Bluetooth technology, a request signal for pairing the user terminal and the data reception device may be transmitted and received. If both terminals have a history of being paired before, both terminals may be automatically connected when they come within the communicable distance. As another example, when an NFC tag is used, if the user terminal and the data reception device approach within a distance where NFC signals may be transmitted and received, a diabetic patient may be identified based on the identified NFC signal.

In a similar manner, user identification may be performed using a known short-range communication technology (QR code, RFID, P2P, etc.). The information transmitted and received in the above process corresponds to communication identification information, and the serial number, licensing key, etc., of the user terminal may be unique information of the user terminal.

The data reception device 200 according to an embodiment of the present disclosure may specify medical data to be requested using the first user identification information.

According to an embodiment of the present disclosure, the data reception device 200 may request preset data among the medical data of the first user collected from the user terminal 100 (S405).

At this time, the preset data may correspond to data specified by the second user of the data reception device based on the first user identification information.

Specifically, the data specified by the second user may differ depending on whether the first user is a general user, a subject of examination, or a patient requiring periodic monitoring (hypertension, diabetes).

Specifically, patients requiring monitoring for hypertension or diabetes may have similar typical symptoms caused by the corresponding diseases, but may have different medical data to be monitored depending on their physical condition, constitution, and other environmental factors, and this may be determined based on the first user identification information.

The second user of the data reception device 200 may provide personalized medical services by specifying the data to be received by the data reception device among multiple medical data held by the user terminal based on the first user identification information.

Meanwhile, the data reception device is preferably placed in a medical institution or a location for collecting medical data, and the second user is preferably a person who has a certain level of medical knowledge on diabetes treatment and diagnosis, such as the first user's diet, medication guidance, and prescriptions, and is preferably a doctor, nurse, or a worker at a medical institution.

As described above, the second user of the data reception device 200 having the specialized knowledge may specify in advance the medical data to be received from the user terminal among the medical data based on the first user identification information.

Hereinafter, an example of the medical data specified based on the first user identification information will be described.

The 'preset data' according to an embodiment of the present disclosure may be set based on diagnostic information of the identified first user. At this time, the diagnostic information may be information stored in the data reception device 200 or information received from an electronic medical record (EMR) connected to the data reception device 200.

For example, when the identification information of the first user among a plurality of users is received and the diagnostic information corresponding to the first user identification information is 'average blood sugar monitoring required', the medical data preset by the second user of the data reception device 200 may include at least one of the maximum blood sugar, the minimum blood sugar, the average blood sugar, and the blood sugar graph among the blood sugar measurement information of the first user.

Through this, the second user of the data reception device may immediately check the maximum blood sugar, minimum blood sugar, and average blood sugar data of the first user collected by the user terminal after the previous medical institution visit of the first user, thereby taking appropriate diagnosis and measures for the first user.

As another example, when the first user identification information is received from among a plurality of users, and the diagnostic information corresponding to the first user identification information is 'specific time blood sugar monitoring required', the medical data preset by the second user of the data reception device may include at least one of the fasting blood sugar, the specific time blood sugar information, the pre-meal blood sugar information, the post-meal blood sugar information, and the pre- and post-exercise blood sugar information among the blood sugar measurement information of the first user.

Similarly, the second user of the data reception device may use the blood sugar data at specific points in time, such as the pre- and post-meal blood sugar and fasting blood sugar of the first user collected by the user terminal after the previous medical institution visit of the first user, to take appropriate diagnosis and measures for the first user.

According to an embodiment of the present disclosure, when the first user identification information among a plurality of users is received, and the diagnostic information corresponding to the first user identification information is 'blood pressure or body temperature monitoring required', the medical data preset by the second user of the data reception device may include at least one of blood pressure, body temperature, oxygen saturation, and heart rate among the health information of the first user.

According to the embodiment, the second user of the data reception device may use the health information of the first user collected by the user terminal after the previous examination or medical institution visit of the first user to take appropriate diagnosis and measures for the first user.

Meanwhile, according to the embodiment of the present disclosure, the 'preset data' among the medical data may be provided in various categories and classifications, and is not limited to the above examples.

In addition, if the second user of the data reception device wants to obtain additional data not included in the preset medical data, the second user may request additional medical data from the user terminal through the communication part of the data reception device, and the user terminal may provide data corresponding to the request received from the data reception device to the data reception device.

Through the above process, it will be known that the preset medical data is set based on the first user identification information and the diagnostic information of the identified first user.

FIG. 4 will be explained again.

The user terminal according to an embodiment of the present disclosure may transmit data corresponding to preset specific data among the medical data of the first user to the data reception device (S407).

The data reception device according to an embodiment of the present disclosure may, after receiving the preset data among the medical data of the first user, output the received medical data (S409).

Thereafter, the second user of the data reception device may input additional diagnostic information of the first user using the output medical data. The data reception device may update the preset specific data based on the additional diagnostic information of the first user input by the second user.

For example, the previous preset data may be 'specific time blood sugar monitoring required', and the preset data updated based on the subsequent diagnostic information may be 'specific time and average blood sugar monitoring required'.

In other words, when the user terminal is connected to the data reception device subsequently, the user terminal may transmit at least one or more data among fasting blood sugar, specific time blood sugar information, pre-meal blood sugar information, post-meal blood sugar information, pre- and post-exercise blood sugar information, maximum blood sugar, minimum blood sugar, average blood sugar, and the blood sugar graph to the data reception device.

The medical data transmission system of the present disclosure may effectively output advanced data for treatment of each patient by updating the 'preset data' set by the data reception device as described above.

Hereinafter, a medical data measuring method, measuring means, and data storage method of the user terminal according to an embodiment of the present disclosure will be described in detail.

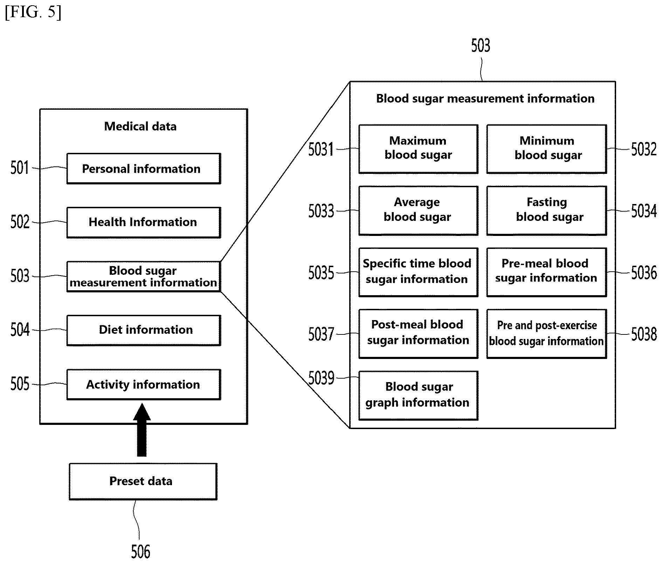

FIG. 5 is a diagram illustrating a method of storing medical data of a user terminal according to an embodiment of the present disclosure.

The user terminal according to an embodiment of the present disclosure may separately manage medical data of the first user by using an application or its own function provided therein.

Referring to FIG. 5, the user terminal may include personal information 501 including at least one of the age, gender, and name of the first user, health information 502 including at least one of blood pressure, body temperature, oxygen saturation, and heart rate, and blood sugar measurement information 503 including at least one of maximum blood sugar, minimum blood sugar, average blood sugar, fasting blood sugar, specific time blood sugar information, pre-meal blood sugar information, post-meal blood sugar information, pre- and post-exercise blood sugar information, and a blood sugar graph according to a blood sugar monitoring method.

At this time, the personal information may also be included in user identification information.

In addition, it may include diet information 504 input by the user, and activity information 505 that records the exercise results of the first user.

When the user terminal is connected to the data reception device, the user terminal may receive data 506 preset by the corresponding data reception device and transmit medical data corresponding to the specific data 506 to the data reception device.

The user terminal may set each data illustrated in FIG. 5 into a major category and number each minor category information included in the major category.

Referring to FIG. 5, the major categories may correspond to personal information 501, health information 502, blood sugar measurement information 503, diet information 504, and activity information 505. The minor categories may refer to detailed information included in the major categories.

For example, blood sugar measurement information 503 may be set as a major category, and each of the maximum blood sugar 5031, minimum blood sugar 5032, average blood sugar 5033, fasting blood sugar 5034, specific time blood sugar information 5035, pre-meal blood sugar information 5036, post-meal blood sugar information 5037, pre- and post-exercise blood sugar information 5038, and blood sugar graph information 5039 included in the blood sugar measurement information 5033 may be numbered as a minor category.

When the user terminal is connected to a data receiving device, the user terminal may obtain major category and minor category numbering information included in the preset data 506, and transmit information corresponding to the obtained minor category numbering to the data reception device.

At this time, the major category and minor category numbering information may be determined in advance based on diagnostic information included in decision element information 507.

In other words, if the diagnostic information of the identified user included in the 'decision element information' is 'average blood sugar monitoring required', the preset data 506 may have a major category of blood sugar measurement information 503 and the minor category numbering information may correspond to '5031' and '5032', and the maximum blood sugar 5031 and minimum blood sugar 5032 information corresponding to the corresponding numbering may be transmitted to the data reception device.

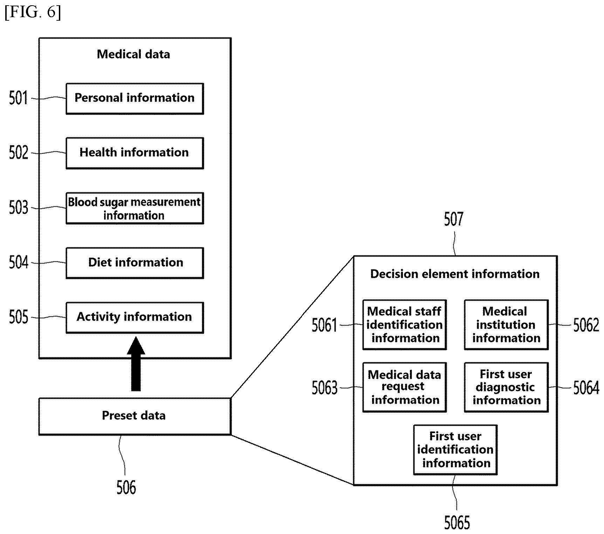

FIG. 6 is a diagram illustrating data for determining preset specific data according to an embodiment of the present disclosure.

Referring to FIG. 6, the decision element information 507 may include information for requesting preset data from a connected data reception device when a user terminal and a data connected device are connected.

In the foregoing, the first user was identified through user identification information by short-range communication, and preset medical data was determined through diagnostic information 5064 of the identified first user.

According to an embodiment of the present disclosure, a user using the data reception device may also be identified, and requested medical data may also be set differently depending on the user using the data reception device.

The decision element information may include at least one of first user identification information, second user identification information, medical institution information, first user diagnostic information, and medical data request information, and may be a database for generating preset data 506.

Specifically, the preset data 506 requested from the data reception device may correspond to data specified by the second user of the data reception device based on the diagnostic information corresponding to the first user identification information and the second user identification information of the data reception device.

At this time, the second user identification information of the data reception device means information for identifying the second user using the data reception device, and may include at least one of medical staff identification information 5061 using the data reception device, medical institution information 5062 providing medical services, and medical data request information 5063 additionally requested by a specific medical staff and a specific medical institution among the medical data.

Through this, the preset data may include the diagnostic information 5064 that diagnosed the first user identified by the medical service providing institution, and may be generated by further considering at least one of the medical staff dentification information 5061 that provides medical services to the first user, medical institution information 5062 providing medical services, and medical data request information 5063.

Hereinafter, a scenario in which the second user identification information of the data reception device is input will be described.

According to an embodiment of the present disclosure, the second user of the data reception device may input medical staff identification information, medical institution information, and medical data request information into the data reception device to generate 'preset data' based on the second user identification information of the data reception device and the diagnostic information 5064 of the identified first user when using the data reception device.

The data reception device may receive the medical staff identification information, medical institution information, and medical data request information, and generate 'preset data' including information additionally requested from the decision element information according to the diagnostic information of the identified first user. The user terminal may, when connected to the data reception device, receive the preset data information and transmit the medical data included in the preset data to the data reception device.

Through this, a personalized medical data transmission system may be provided not only to the first user but also to the second user of the data reception device who provides medical services.

Hereinafter, a measurement means and a measurement method of blood sugar measurement information will be described.

The user terminal according to an embodiment of the present disclosure may measure a blood sugar level of a diabetic patient in real time using a blood sugar measurement part included in a sensing part, and generate blood sugar monitoring data based on the measured blood sugar level.

In addition, the user terminal may communicate with a separate blood sugar measurement device and measure the blood sugar level of a diabetic patient in real time through the blood sugar measurement sensor equipped in the blood sugar measurement device. Since the above two embodiments are a problem of whether the blood sugar measurement device corresponds to a configuration equipped inside the user terminal or is distinguished as a blood sugar measurement device that exists separately externally, both should be interpreted as being included in the embodiments of the present disclosure.

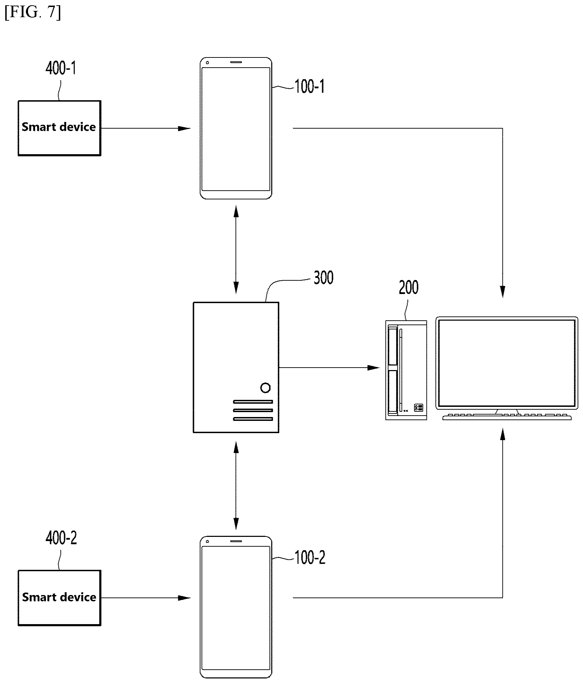

Hereinafter, a system of a scenario in which a separate blood sugar measurement device exists will be described with reference to FIG. 7.

FIG. 7 illustrates another embodiment of a medical data transmission system according to an embodiment of the present disclosure.

Referring to FIG. 7, a medical data transmission system for transmitting and receiving medical data of a first user according to an embodiment of the present disclosure may include at least one user terminal 100-1, 100-2, a data reception device 200, a server 300, and smart devices 400-1, 400-2 connected to each of the user terminals.

Specifically, the smart devices connected to each of the user terminals may include a wearable device capable of obtaining first user biosignal information or a blood sugar measurement device capable of measuring the blood sugar level of the first user.

In the case of a wearable device, it may include the configuration of FIG. 1, just like the user terminal. In addition, in the case of a blood sugar measurement device, the blood sugar measurement device may include a communication part for communicating with the user terminal, a blood sugar measurement part for measuring blood sugar information of the diabetic patient, and a data generation part for generating blood sugar data based on the type of the blood sugar measurement device.

Hereinafter, the wearable device and the blood sugar measurement device are collectively referred to as smart devices.

The user terminals 100-1, 100-2 may be linked with the smart devices 400-1, 400-2 respectively connected thereto to obtain medical data.

The smart devices 400-1, 400-2 according to an embodiment of the present disclosure may obtain blood sugar information and biometric data of the first user using known technologies each possesses, and may transmit the obtained medical data to the user terminal using a wireless communication network, a self-installed application, Bluetooth, an NFC tag, etc.

The user terminal may store medical data received from each of the smart devices 400-1, 400-2 and transmit the collected medical data to the server 300. In addition, the user terminal may, when approaching the data reception device 200 within a predetermined distance, transmit specific data among the collected medical data to the data reception device 200.

Matters related to the remaining operations are the same as those described in FIGS. 1 to 6 of the present disclosure.

It may be seen that the medical data transmitted to the server through the embodiment to be implemented is different from the preset medical data transmitted to the data reception device, and rather than having a plurality of users for the user terminal, since each of the plurality of individuals is equipped with it, the user of the data reception device will be able to provide personalized medical services by utilizing the personal medical data.

Through this, when medical data corresponding to the first user identification information is requested from the data reception device, the data reception device may output the medical data without a separate data processing process by transmitting the data stored in the user terminal as it is, and advanced and effective treatment of diabetes patients may be performed.

The present disclosure described above may be implemented as a computer-readable code on a medium in which a program is recorded. Non-transitory computer-readable media include any type of recording device that stores data that may be read by a computer system. Examples of non-transitory computer-readable media include hard disk drive (HDD), solid state disk (SSD), silicon disk drive (SDD), ROM, RAM, CD-ROM, magnetic tape, floppy disk, and optical data storage device.

## Claims

1. A medical data transmission system for transmitting and receiving medical data, the system comprising:
a user terminal configured to collect first user medical data and transmit first user identification information; and
a data reception device configured to identify a first user based on the first user identification information received from the user terminal, receive preset data based on the first user identification information among the first user medical data collected from the user terminal, and output the preset data.

2. The system of claim 1, wherein the first user identification information comprises personal information comprising at least one of age, gender, and name of the first user.

3. The system of claim 1, wherein the first user medical data comprises health information comprising at least one of blood pressure, body temperature, oxygen saturation, and heart rate, and blood sugar measurement information comprising at least one of maximum blood sugar, minimum blood sugar, average blood sugar, fasting blood sugar, specific time blood sugar information, pre-meal blood sugar information, post-meal blood sugar information, pre- and post-exercise blood sugar information, and a blood sugar graph according to a blood sugar monitoring method of the first user.

4. The system of claim 1, wherein the preset data is set based on diagnostic information corresponding to the first user identification information.

5. The system of claim 4, wherein, when the diagnostic information is 'average blood sugar monitoring required',
the preset data comprises at least one of maximum blood sugar, minimum blood sugar, average blood sugar, and a blood sugar graph among blood sugar measurement information of the first user.

6. The system of claim 4, wherein, when the diagnostic information is 'specific time blood sugar monitoring required',
the preset data comprises at least one of fasting blood sugar, blood sugar information at a specific time, pre-meal blood sugar information, post-meal blood sugar information, and pre- and post-exercise blood sugar information among blood sugar measurement information of the first user.

7. The system of claim 4, wherein, when the diagnostic information is 'blood pressure or body temperature monitoring required',
the preset data comprises at least one information of blood pressure, body temperature, oxygen saturation, and heart rate among health information of the first user.

8. The system of claim 4, wherein the data reception device is configured to update the preset data based on additional diagnostic information of the first user.

9. The system of claim 1, wherein the data reception device is configured to, when a distance from the user terminal is within a predetermined distance, communicate with the user terminal and identify the first user using any one of QR authentication, Wi-Fi, Bluetooth, P2P, NFC, and RFID.

10. The system of claim 1, further comprising:
a server connected to the user terminal,
wherein the user terminal is configured to transmit the first user medical data to the server, and the first user medical data transmitted to the server and the preset data are different from each other.

11. The system of claim 1, wherein the preset data corresponds to data specified by a second user of the data reception device based on the first user identification information, first user diagnostic information, and second user identification information of the data reception device.

12. The system of claim 11, wherein the second user identification information comprises at least one of identification information on a medical staff using the data reception device, information on a medical institution providing medical services, and medical data request information additionally requested by a specific medical staff and a specific medical institution among the medical data.

13. The system of claim 1, further comprising:
a blood sugar measurement device configured to measure the first user medical data and transmit the first user medical data to the user terminal,
wherein the blood sugar measurement device comprises a communication part for communicating with the user terminal, a blood sugar measurement part configured to measure blood sugar information of the first user, and a data generation part configured to generate data based on a type of the blood sugar measurement device.

14. A medical data transmission method for transmitting and receiving user medical data, the method comprising:
by a user terminal, communicating with a data reception device and collecting first user medical data;
by the data reception device, identifying, the user terminal and a first user and receiving preset data based on first user identification information among the first user medical data collected from the user terminal; and
outputting, by the data reception device, the preset data.

15. The method of claim 14, wherein, when diagnostic information is 'average blood sugar monitoring required',
the preset data is set based on diagnostic information corresponding to the first user identification information, and
comprises at least one of maximum blood sugar, minimum blood sugar, average blood sugar, and a blood sugar graph among blood sugar measurement information of the first user.

16. The method of claim 14, wherein, when diagnostic information is 'specific time blood sugar monitoring required',
the preset data is set based on diagnostic information corresponding to the first user identification information, and
comprises at least one of fasting blood sugar, specific time blood sugar information, pre-meal blood sugar information, post-meal blood sugar information, and pre- and post-exercise blood sugar information of the first user.

17. The method of claim 14, further comprising updating, by the data reception device, the preset data based on additional diagnostic information of the first user.

18. The method of claim 14, wherein the preset data corresponds to data specified by a second user of the data reception device based on the first user identification information, first user diagnostic information, and second user identification information of the data reception device, and
the second user identification information of the data reception device comprises at least one of identification information on a medical staff using the data reception device, information on a medical institution providing medical services, and medical data request information additionally requested by a specific medical staff and a specific medical institution among the medical data.
